Europäisches Patentamt

European Patent Office

Office européen des brevets

(19)

(11) Publication number : **0 496 162 A2**

(12)

# EUROPEAN PATENT APPLICATION

(21) Application number : **91311658.8**

(51) Int. Cl.⁵ : **C12N 15/12, C07K 13/00**

(22) Date of filing : **16.12.91**

(30) Priority : **24.12.90 US 632891**

(43) Date of publication of application :
**29.07.92 Bulletin 92/31**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Schaber, Michael D.**
**839 Price Road**
**Harleysville, PA 19438 (US)**

Inventor : **Garsky, Victor M.**
**572 Palmer Drive**
**Bluebell, PA 19422 (US)**
Inventor : **Gibbs, Jackson B.**
**118 Tartan Terrace**
**Chalfont, PA 18914 (US)**
Inventor : **Sigal, Irving S.**
**45 Vandeventer C-2**
**Princeton, NJ 08542 (US)**
Inventor : **Freidinger, Roger M.**
**744 Newport Lane**
**Lansdale, PA 19446 (US)**

(74) Representative : **Thompson, John Dr.**
**Merck & Co., Inc. European Patent**
**Department Terlings Park Eastwick Road**
**Harlow, Essex CM20 2QR (GB)**

(54) **Peptide inhibitors of RAS-GAP interaction.**

(57)    Oncogenic forms of Ras protein have been reported to occur in a variety of human cancers, including colorectal carcinoma, exocrine pancreatic carcinoma, and myeloid leukemias. Active forms of Ras protein which are complexed with GTP can bind to GTPase activating protein (GAP). This binding is thought to be an important step in the cell transforming activity of Ras. According to the present invention a group of peptides have been discovered which block the binding of Ras to GAP.

EP 0 496 162 A2

## BACKGROUND OF THE INVENTION

The product of the Harvey (Ha) ras oncogene is a 21 kilodalton (kDa) membrane-bound protein which binds GTP and GDP, and has an intrinsic GTPase activity. The ras oncogene product is referred to as p21 or Ras. Ras has structural similarities with other regulatory G proteins, and is biochemically and biologically active only when complexed with GTP. Specific amino acid changes in Ras at positions 12, 13, 59, 61 and 63 impair the intrinsic GTPase activity, thereby stabilizing the protein in the active GTP form. These oncogenic mutations of ras have been reported to occur in a variety of neoplasia, including human colorectal tumors, carcinomas from human exocrine pancreas, and myeloid leukemias.

In yeast Saccharomyces cerevisae, RAS proteins regulate the levels of intracellular cyclic AMP (cAMP) by stimulating adenylyl cyclase activity. This interaction occurs only with RAS-GTP, and not with RAS-GDP. Biochemical evidence has indicated that adenylyl cyclase is not the Ras target in mammalian cells.

Critical to the understanding of Ras function has been the analysis of the structure of this 189 amino acid oncogene product. Prior to the solution of the crystal structure of Ras, mutagenesis studies of Ras identified regions of the protein involved with membrane localization, guanine nucleotide binding, binding of a neutralizing antibody (nAb), and interaction with target proteins. Membrane localization is essential for Ras function in mammalian cells and involves Cys-186. The purine ring of GDP interacts with Asn-116 and Asp-119, and the phosphates lie near residues 12-16. The residues that comprise the nAb epitope are Glu-63, Ser-65, Ala-66, Met-67, Gln-70, and Arg-73.

Microinjection of Ras neutralizing antibody Y13-259, into mammalian cells or Xenopus oocytes inhibits DNA synthesis and morphological changes induced by Ras. Although Y13-259 neutralized Ras function, mutations within the epitope reacting with Y13-259, do not impair Ras function, indicating that the Y13-259 acts indirectly. The putative effector region is a surface domain of Ras spanning amino acids 32-40 that is critical for the interaction between Ras and its target. Mutations in this region impair the cell transforming activity of Ras, but are without effect on membrane localization, or GDP binding.

The region 32-40 could either be a direct point of interaction between Ras and its target, or might influence the adoption of an active conformation.

Studies of the ras oncogene are directed toward identifying the Ras target in mammalian cells and understanding the mechanisms that regulate Ras activity. Recently, the GTPase activating protein (GAP) was described by Trahey and McCormick (Science (1987), 238, pp.542-545) as an activity in the cytosolic fraction of Xenopus oocytes which was able to stimulate the intrinsic GTPase activity of normal Ras 100-fold, but was without effect on the impaired GTPase activity of oncogenic Ras. Through its interaction with Ras, GAP may be acting as an upstream negative regulator of Ras, by affecting the biochemical activity which determines the biological potency of the protein. Alternatively, GAP may act as a downstream target of Ras, in a manner analogous to the interaction between EF-Tu and the ribosome. In higher eukaryotic organisms such as mammals and Xenopus, only GAP has been shown to interact with Ras.

In order to evaluate GAP function in the Ras pathway, bovine GAp was purified as a monomeric 125 kDa polypeptide and the cDNA was cloned. The GAP gene encodes a protein of 1044 amino acids. Although purified GAP does not stimulate the GTPase activity of oncogenic Ras mutants, it is able to physically bind to the GTP forms of both normal and oncogenic Ras in vitro. Mutations in the Ras region 32-40 that impair biological function can also inhibit binding to GAP, raising the possibility that GAP might be the immediate target of Ras. The Ras structural requirements for binding to GAP in vitro are similar to those needed for stimulation of S. cerevisiae adenylyl cyclase activity, an effector enzyme of Ras.

The sequence of GAP has shown a significant amino acid similarity between regions in the N-terminal half of GAP and the sequences of B and C boxes, which are conserved among the noncatalytic domains of nonreceptor tyrosine kinase oncogene products, the crk oncogene product and phospholipase C-γ. By deletion mutagenesis, a 343 amino acid C-terminal fragment of GAP ([702-1044]GAP) was identified which has the same catalytic activity and Ras binding affinity, as full length GAP purified from bovine brain. In the C-terminal domain, GAP shares amino acid similarity at residues 896 to 948 with the S. cerevisiae gene product IRA which interacts with yeast Ras. The amino acid similarity between GAP and IRA may represent a Ras binding domain [Tanaka, K. et al., (1989) Mol. Cell Biol., 9, pp. 757-768]. Together, these observations suggest that GAP may have two structural domains, with the N-terminus having a functional role distinct from the C-terminal Ras binding domain.

## OBJECTS OF THE INVENTION

It is an object of the present invention to provide peptides that inhibit the interaction of Ras with GAP. Another object of the present invention is to provide peptides that inhibit the interaction of Ras with yeast adenylyl cyclase. An additional object of the present invention is to provide peptides which block the biological activity of Ras protein. These and other objects of the present invention will be apparent from the following description.

## SUMMARY OF THE INVENTION

A group of synthetic peptides have been found to inhibit the interaction of the 189 amino acid Harvey ras oncogene product (Ras) with its cellular targets. The peptides of the present invention correspond to the region of the Ras protein which encompasses amino acids 17 to 44. These peptides inhibit the interaction of Ras with the GTPase activating protein (GAP) as well as the interaction of Ras with yeast adenylyl cyclase. The greatest inhibitory potency was exhibited by a peptide which corresponds to amino acids 19 to 32 of Ras.

## DETAILED DESCRIPTION OF THE INVENTION

The activity of the synthetic peptides of the present invention, as inhibitors of the interaction of ras with GAP was determined in an assay which measures GAP dependent, Ras GTPase activity. Bovine GAP was either purified from fresh bovine brains or recombinantly produced in E. coli. It is understood that the methods of the present invention may utilize either the recombinant full length GAP or the bovine brain derived GAP. In addition, GAP from other species, such as human and mouse, may be substituted for bovine GAP since they share about 95% amino acid homology. Truncated recombinant forms of GAP are also suitable for use in the Ras-GAP interaction assay [Marshall, M.S., et al., (1989), EMBO J., 8, pp1105-1110]. However, for the screening of synthetic peptides for their ability to block the Ras-GAP interaction, the recombinant, full length GAP is preferred.

The Ras protein utilized in the Ras-GAP GTPase assay of the present invention is Harvey ras (Ha-ras). It is understood that other forms of Ras protein are suitable for use in the present invention. Other suitable forms of Ras protein include, but are not limited to, Kirsten Ras, yeast S. cerevisiae RAS, and N-Ras. Furthermore, it is readily apparent to those skilled in the art that any form of Ras protein which is capable of interacting with GAP is suitable for use in the Ras-GAP GTPase assay.

The Ras-GAP GTPase assay was utilized to test the ability of the synthetic peptides of the present invention, to block Ras interaction with GAP. The use of this assay system identified the synthetic peptides which possess the desired property of inhibiting the function of Ras, with respect to GAP.

An additional assay system, using yeast S. cerevisiae strains MY1186, JC377-10B [Gibbs, J. et al., (1988) Bioch. J.,252, pp289-292] and FID [DeVendittis, E. et al., (1986), EMBO J., 5, pp. 3657-3664] adenylyl cyclase reconstitution, was utilized for the purpose of confirming the results obtained from the Ras-GAP binding inhibition assay, as well as demonstrating the ability of the synthetic peptides of the present invention, to inhibit Ras function in an analogous system from a lower eukaryotic organism. S. cerevisiae strain MY1186 lacks endogenous yeast Ras, expresses viral Ha-Ras, and has a Ras-dependent adenylyl cyclase. Strain JC377-10B lacks Ras and has a mutant adenylyl cyclase that is Ras-independent. S. cerevisiae strain FID lacks Ras protein in the cell membrane, yet the cell membranes contain adenylyl cyclase which can be reconstituted with purified Ras. By measuring the Ras-activated conversion of ATP into cyclic AMP using exogenous purified Ras, the ability of the synthetic peptides of the present invention to inhibit Ras function was reaffirmed.

Ras has been found to be associated with certain types of tumors in humans. Furthermore, Ras has been shown to transform mammalian cells in culture, following microinjection or expression of Ras protein from a DNA expression plasmid. Another means of demonstrating transformation by Ras protein is the induction of germinal vessicle breakdown (GVBD) in Xenopus oocytes following microinjection of Ras protein. In Xenopus oocytes, microinjection of antibodies which neutralize Ras function blocks GVBD induced by insulin, but not GVBD induced by progesterone. GVBD causes the appearance of a white spot in the animal hemisphere of the oocyte.

The biological activity of the synthetic peptides of the present invention as inhibitors of Ras function was determined in Xenopus oocyte GVBD assays. Because Xenopus oocytes respond to Ras microinjection by inducing GVBD (a widely accepted model of transformation), peptides that block Ras-GAP interactions can be tested in this system for their ability to inhibit Ras-induced transformation. Cellular transformation is an early step in tumor formation. Compounds which block cell transformation are useful as tools for understanding the mechanisms of transformation as well as potential inhibitors of tumor formation.

The efficacy of the synthetic peptides of the present invention as inhibitors of Ras-GAP interaction and Ras-mediated cell transformation, was demonstrated by measuring both the inhibition of GAP-dependent Ras GTPase, and Ras-mediated Xenopus oocyte GVBD. The peptides, peptide subunits, and peptide derivatives of the present invention can be synthesized from their constituent amino acids by conventional peptide synth-

EP 0 496 162 A2

esis techniques, preferably by solid-phase technology. The peptides are then purified by reverse-phase high performance liquid chromatography (HPLC).

Standard methods of peptide synthesis are disclosed, for example, in the following works: Schroeder et al., "The Peptides", Vol. I, Academic Press 1965, or Bodanszky et al., "Peptide Synthesis", Interscience Publishers, 1966, or McOmie (ed.) "Protective Groups in Organic Chemistry", Plenum Press, 1973, or Barany et al., "The Peptides: Analysis, Synthesis, Biology" 2, Chapter 1, Academic Press, 1980. The teachings of these works are hereby incorporated by reference.

The peptides of the present invention are related to the contiguous amino acids of Ras protein corresponding to Ras amino acids 17 through 44, and subunits thereof, having the formula:

```
Ser Ala Leu Thr Ile Gln Leu Ile Gln Asn His
1               5                   10
Phe Val Asp Glu Tyr Asp Pro Thr Ile Glu Asp Ser
                15                  20
Tyr Arg Lys Gln Val.
    25
```

A peptide was synthesized which corresponds to this amino acid sequence, and was designated Ras 17-44 (SEQ ID NO:1). Another peptide was synthesized which corresponds to the portion of GAP that interacts with Ras, except that the amino acid Gly was substituted for Cys at residue 903 of GAP. The substitution of Gly for Cys was done to prevent disulfide bond formation during or following peptide synthesis. This peptide was designated GAP 888-910(Gly903)(SEQ ID NO:2), and has the amino acid sequence:

```
Met Arg Thr Arg Val Val Ser Gly Phe Val Phe Leu
1               5                   10
Arg Leu Ile Gly Pro Ala Ile Leu Asn Pro Arg.
    15                  20
```

These peptides were shown to inhibit Ras-GAP interaction in the GAP assay of Example VII. Since the Ras peptide was much more potent, derivatives of Ras 17-44 were also synthesized, which consisted of $CO_2H$-terminal truncations, $NH_2$-terminal truncations, and modifications of specific amino acids. Peptides with $CO_2H$-terminal amino acid substitutions and truncations are shown in Table I, with their respective Ras amino acid designation. Peptide Ras 17-37 is SEQ ID NO:1 from which 7 amino acids have been deleted from the carboxy terminus. Peptide Ras 17-32 is SEQ ID NO:1 from which 12 amino acids have been deleted from the carboxy terminus. Peptide Ras 17-29 is SEQ ID NO:1 from which 15 amino acids have been deleted from the carboxy terminus. Peptide Ras 17-26 is SEQ ID NO:1 from which 18 amino acids have been deleted from the carboxy terminus.

4

## TABLE I

### CO$_2$H-terminal Ras 17-44 truncations

| Ras Amino Acid Designation | Peptide Sequence |
|---|---|
| Ras 17-37 | Ser Ala Leu Thr Ile Gln Leu<br>1          5<br>Ile Gln Asn His Phe Val Asp<br>       10<br>Glu Tyr Asp Pro Thr Ile Glu<br>15          20 |
| Ras 17-32 | Ser Ala Leu Thr Ile Gln Leu<br>1          5<br>Ile Gln Asn His Phe Val Asp<br>       10<br>Glu Tyr<br>15 |
| Ras 17-29 | Ser Ala Leu Thr Ile Gln Leu<br>1          5<br>Ile Gln Asn His Phe Val<br>       10 |
| Ras 17-26 | Ser Ala Leu Thr Ile Gln Leu<br>1          5<br>Ile Gln Asn<br>       10 |

These CO$_2$H-terminal modified or truncated peptides were then tested in the Ras-GAP interaction inhibition assay (described in Example VII) for their ability to block GAP stimulated Ras GTPase activity Table II shows the results of the Ras-GAP assay performed using the CO$_2$H-terminal truncated peptides. Also shown in Table II are the maximum solubility concentrations for each peptide. All assays were performed using fully soluble peptide solutions, according to Example VII.

## TABLE II

### Ras-GAP Assay

| Peptide | $IC_{50}*$ $\mu M$ | Maximum Solubility $(\mu M)$[1] |
|---|---|---|
| GAP 888-910(Gly903) (SEQ ID NO:2) | 100 | 800 |
| Ras 17-44 (SEQ ID NO:1) | 2.4 | 200 |
| Ras 17-37 | 3.6 | 40 |
| Ras 17-32 | 0.9 | 19 |
| Ras 17-29 | no inhibition | 20 |
| Ras 17-26 | no inhibition | 600 |

$*-IC_{50}$ is defined as the concentration of peptide required to reduce GAP stimulated Ras-GTP hydrolysis by 50%.

1-maximum solubility refers to aqueous solutions having 20% DMF.

The results of this assay identified three peptides, Ras 17-44 (SEQ ID NO:1), Ras 17-37, and Ras 17-32, which had Ras-GAP inhibitory properties. The shortest $CO_2H$-terminal truncated peptide which maintained inhibitory activity was Ras 17-32. These peptides did not effect intrinsic Ras GTPase activity, assayed in the absence of GAP, indicating that they inhibited Ras-GAP interaction.

In addition, Ras 17-32 was the most potent Ras-GAP inhibitor demonstrated by the lowest $IC_{50}$ of all $CO_2H$-terminal truncated peptides.

The amino acid sequence of Ras 17-32 was the basis for synthesizing a series of $NH_2$-terminal truncated peptides. In addition, peptides were synthesized which correspond to $NH_2$-terminal deletions of Ras 17-44. These $NH_2$-terminal truncated peptides are shown in Table III, with their respective Ras amino acid designation.

Peptide Ras 19-31 is SEQ ID NO:1 from which 13 amino acids have been deleted from the carboxy terminus and from which two amino acids have been deleted from the amino terminus. Peptide Ras 19-32 is SEQ ID NO:1 from which 12 amino acids have been deleted from the carboxy terminus and from which two amino acids have been deleted from the amino terminus. Peptide Ras 19-33 is SEQ ID NO:1 from which 11 amino acids have been deleted from the carboxy terminus and from which two amino acids have been deleted from the amino terminus. Peptide Ras 21-32 is SEQ ID NO:1 from which 12 amino acids have been deleted from the carboxy terminus and from which four amino acids have been deleted from the amino terminus. Peptide Ras 26-32 is SEQ ID NO:1 from which 12 amino acids have been deleted from the carboxy terminus and from which nine amino acids have been deleted from the amino terminus. Peptide Ras 30-32 is SEQ ID NO:1 from which 12 amino acids have been deleted from the carboxy terminus and from which 13 amino acids have been deleted from the amino terminus. Peptide Ras 23-37 is SEQ ID NO:1 from which seven amino acids have been deleted from the carboxy terminus and from which six amino acids have been deleted from the amino terminus. Peptide Ras 31-43 is SEQ ID NO:1 from which one amino acid has been deleted from the carboxy terminus and from which 14 amino acids have been deleted from the amino terminus.

## TABLE III

### NH$_2$-Terminal Ras Peptide Truncations

| Ras Amino Acid Designation | Peptide Sequence |
|---|---|
| Ras 19-31 | Leu Thr Ile Gln Leu Ile Gln Asn His Phe Val Asp Glu |
| Ras 19-32 | Leu Thr Ile Gln Leu Ile Gln Asn His Phe Val Asp Glu Tyr |
| Ras 19-33 | Leu Thr Ile Gln Leu Ile Gln Asn His Phe Val Asp Glu Tyr Asp |
| Ras 21-32 | Ile Gln Leu Ile Gln Asn His Phe Val Asp Glu Tyr |
| Ras 26-32 | Asn His Phe Val Asp Glu Tyr |
| Ras 30-32 | Asp Glu Tyr |

## TABLE III
(continued)

| | |
|---|---|
| Ras 23-37 | Leu Ile Gln Asn His Phe Val Asp Glu Tyr Asp Pro Thr Ile Glu |
| Ras 31-43 | Glu Tyr Asp Pro Thr Ile Glu Asp Ser Tyr Arg Lys Gln |

These NH$_2$-terminal truncated peptides were then tested in the Ras-GAP interaction inhibition assay (described in Example VII) for their ability to block GAP stimulated Ras GTPase activity. Table IV shows the results of the Ras-GAP assay performed using the NH$_2$-terminal truncated peptides. Also shown in Table IV are the maximum solubility concentrations for each peptide.

## TABLE IV

### Ras-GAP Assay

| Peptide | $IC_{50}$* $\mu M$ | Maximum Solubility ($\mu M$)[1] |
|---|---|---|
| Ras 19-31 | 48 | >1000 |
| Ras 19-32 | 0.4 | 48 |
| Ras 19-33 | 1.4 | 23 |
| Ras 20-32 | 18 | 110 |
| Ras 21-32 | 70 | 120 |
| Ras 26-32 | 350 | 800 |
| Ras 30-32 | no inhibition | 800 |

## TABLE IV
(continued)

| | | |
|---|---|---|
| Ras 23-37 | no inhibition | 250 |
| Ras 31-43 | no inhibition | 446 |

*-$IC_{50}$ is defined as the concentration of peptide required to reduce GAP stimulated Ras-GTP hydrolysis by 50%.

1-maximum solubility refers to aqueous solutions having 20% DMF.

The results of this assay identified four additional peptides, Ras 19-32, Ras 20-32, Ras 21-32 and Ras 26-32, which had Ras-GAP inhibitory properties. The most potent $NH_2$-terminal truncated Ras-GAP inhibitory peptide was Ras 19-32.

The results with peptide Ras 26-32 identifies the minimal region of Ras able to interact with GAP. This region may influence the cell transforming activity of Ras as well as the tumor suppressing activity of RaplA/K-rev [Kitayama, H. et al., (1989) Cell, 56 pp77-84] since both proteins have identical sequences in the region 32-40 but are divergent in the region 21-31. Therefore, the identification of the minimal region of Ras capable of GAP interaction, peptide Ras 26-32, indicates the structural importance of this region for cell transformation.

Peptides Ras 19-31, 19-32 and Ras 19-33 were further studied by modification deletion, and addition of specific amino acids. Solubility of a peptide may be increased by adding charged amino acids, such as Lys, Arg, His, Asp, Glu, and ornithine, or derivatives of charged amino acids or deleting uncharged amino acids, such as Leu. Stability of the peptide may be improved by adding blocking groups at the $NH_2$- and/or $CO_2H$-terminus, such as acetyl ($NH_2$-terminus), or amido ($CO_2H$-terminus) groups. The replacement of a naturally occurring L-amino acid with the D-isomer can also improve peptide stability.

These modified peptides are shown in Table V.

## TABLE V

### Modifications of Ras Peptides

| Ras Amino Acid Designation | Peptide Sequence |
|---|---|
| Ras 19-31(1)<br>(SEQ ID NO:3) | Leu Thr Ile Gln Ile Gln Asn His<br>1                  5<br>Phe Val Asp Glu (lacks Leu 23)<br>10 |
| Ras 19-32(D-Asn)<br>(SEQ ID NO:4) | Leu Thr Ile Gln Leu Ile<br>1              5<br>Gln Xaa His Phe Val Asp Glu<br>10<br>Tyr |
| Ras 19-33(1)<br>(SEQ ID NO:5) | Arg Arg Leu Thr Ile Gln Leu Ile<br>1                  5<br>Gln Asn His Phe Val Asp Glu Tyr Xaa<br>10                       15 |
| Ras 19-33(2)<br>(SEQ ID NO:6) | Xaa Arg Leu Thr Ile Gln Leu Ile<br>1                  5<br>Gln Asn His Phe Val Asp Glu Tyr Xaa<br>10                       15 |
| Ras 19-33(3)<br>(SEQ ID NO:7) | Glu Glu Leu Thr Ile Gln Leu Ile<br>1                  5<br>Gln Asn His Phe Val Asp Glu Tyr Xaa<br>10                       15 |

## TABLE V
(continued)

```
Ras 19-33(4)        Arg Arg Leu Thr Ile Gln Leu Ile
(SEQ ID NO:8)       1               5
                    Gln Asn His Phe Val Asp Glu Tyr
                        10                      15
                    Asp


Ras 19-33(5)        Arg Arg Leu Thr Ile Gln Leu Ile
(SEQ ID NO:9)       1               5
                    Gln His Phe Val Asp Glu Tyr Asp
                        10                      15
                    (lacks Asn 26)


Ras 19-33(6)        Arg Ala Leu Thr Ile Gln Leu Ile
(SEQ ID NO:10)      1               5
                    Gln Asn His Phe Val Asp Glu Tyr Asp
                        10                      15


Ras 19-33(7)        Ser Arg Thr Ile Gln Leu Ile Gln
(SEQ ID NO:11)      1               5
                    Asn His Phe Val Asp Glu Tyr Xaa
                        10                      15
                    (lacks Leu 19)
```

Peptide Ras 19-31(1) (SEQ ID NO:3) was synthesized to delete the Leu at Ras position 23. Peptide Ras 19-32(D-Asn) (SEQ. ID NO:4) was synthesized by substituting D-Asn for L-Asn. Peptides Ras 19-33(1) (SEQ ID NO:5), Ras 19-33(2) (SEQ ID NO:6), Ras 19-33(3) (SEQ ID NO:7), Ras 19-33(4) (SEQ ID NO:8), Ras 19-33(5) (SEQ ID NO:9), Ras 19-33(6) (SEQ ID NO:10), and Ras 19-33(7) (SEQ ID NO:11) contain two additional amino acids at the $NH_2$-terminus (see Table V), and peptide Ras 19-33(2) (SEQ ID NO:5) has modified the $\alpha$-amino group with an acetyl (Ac) group, on the amino-terminal Arg, (depicted as Xaa) forming Ac-Arg-. In addition, the $\alpha$-$CO_2H$ group of the carboxy-terminal amino acid Asp (depicted as Xaa) of Ras 19-33(1) (SEQ ID NO:5), Ras 19-33(2) (SEQ ID NO:6), Ras 19-33(3) (SEQ ID NO:7), and Ras 19-33(7) (SEQ ID NO:11) was modified to an amido group, forming -ASP-$NH_2$. Peptide Ras 19-33(3) (SEQ ID NO:7) contains two additional amino acids (Glu-Glu) at the $NH_2$-terminus of Ras 19-33, and has substituted an amido group for the $\alpha$-$CO_2H$ group of the carboxy terminal Asp (depicted as Xaa), forming -Asp-$NH_2$. Peptide Ras 19-33(5) (SEQ ID NO:9) lacks Asn at position 26, and Ras 19-33(7) (SEQ ID NO:11) lacks Leu at position 19.

The modified Ras peptides in Table V were tested in the Ras-GAP interaction inhibition assay for their ability to block GAP stimulated, Ras-related GTPase activity. Table VI shows the results of the Ras-GAP assay, in addition to the maximum solubility concentrations, for each peptide.

## TABLE VI

### Ras-GAP Assay on Modified Peptides

| Peptide | $IC_{50}*$ $\mu M$ | Maximum Solubility |
|---|---|---|
| Ras 19-31(1) (SEQ ID NO:3) | 36.0 | >1mM |
| Ras 19-32(D-Asn) (SEQ ID NO:4) | 0.2 | 12μM |
| Ras 19-33(1) (SEQ ID NO:5) | 45.0 | 4mM |
| Ras 19-33(2) (SEQ ID NO:6) | 30.0 | 4mM |
| Ras 19-33(3) (SEQ ID NO:7) | 50.0 | 225μM |
| Ras 19-33(4) (SEQ ID NO:8) | 4.0 | >2mM |
| Ras 19-33(5) (SEQ ID NO:9) | 30.0 | >1mM |
| Ras 19-33(6) (SEQ ID NO:10) | 3.5 | >2mM |
| Ras 19-33(7) (SEQ ID NO:11) | 24.0 | >2mM |

Peptides Ras 19-33(1) and Ras 19-33(2) exhibited greatly enhanced solubility but reduced $IC_{50}$, compared with Ras 19-33. For peptides Ras 19-33(1), Ras 19-33(2), Ras 19-33(4), Ras 19-33(5), Ras 19-33(6), and Ras 19-33(7) these solubilities can also be achieved in aqueous solutions lacking organic solvents, such as DMF. Peptide Ras 19-32(D-Asn) was the most potent of the peptides tested in Ras-GAP assay.

A series of Ras peptides were synthesized for use as negative control (inactive) peptides, which correspond to Ras amino acid sequences which are not thought to be involved with Ras-GAP interactions. The amino acid sequence of these peptides is shown in Table VII. None of these peptides exhibited an inhibitory activity in the Ras-GAP assay. The solubility of these peptides was determined to be within the range of between about 85 μM and about 400 μM.

## TABLE VII

### Negative Control Peptides

| Ras Amino Acid Designation | Peptide Sequence |
|---|---|
| Ras 40-55 (SEQ ID NO:12) | Tyr Arg Lys Gln Val Val Ile Asp<br>1                5<br>GLY Glu Thr Ser Leu Leu Asp Ile<br>    10                    15 |
| Ras 60-76 (SEQ ID NO:13) | Gly Gln Glu Glu Tyr Ser Ala Met<br>1                5<br>Arg Asp Gln Tyr Met Arg Thr Gly<br>    10                    15<br>Glu |
| Ras 91-103 (SEQ ID NO:14) | Glu Asp Ile His Gln Tyr Arg Glu<br>1                5<br>Gln Ile Lys Arg Val<br>    10 |
| Ras 126-137 (SEQ ID NO:15) | Glu Ser Arg Gln Ala Gln Asp Leu<br>1                5<br>Ala Arg Ser Tyr<br>    10 |

Another Ras-dependent enzymatic assay was used to extend the observed ability of the peptides of the present invention to inhibit Ras function. This system, yeast adenylyl cyclase assay, measures the Ras-dependent conversion of ATP into cyclic AMP (cAMP). Yeast cells do not possess GAP in the cell membrane. Furthermore, in S. cerevisiae, regulation of cAMP metabolism appears to be a major function of Ras. This assay system was used to extend the observed Ras inhibitory activity of the peptides of the present invention, to a Ras function which is GAP-unrelated. S. cerevisiae strain FID lacks functional Ras proteins, however FID cells have functional adenylyl cyclase in their cell membrane. Ras-dependent adenylyl cyclase activity can be demonstrated in isolated FID membranes, by adding Ras protein to the membranes. Similarly, a recombinant S. cerevisiae strain MY1186, lacks yeast Ras proteins but expresses mammalian Ras protein which functions with adenylyl cyclase to regulate cAMP metabolisim in the yeast cells. Cell membranes isolated from MY1186 can be used to demonstrate Ras dependent adenylyl cyclase activity. In contrast, S. cerevisiae strain JC377-10B contains a mutant form of adenylyl cyclase which constitutively converts ATP into cAMP with or without Ras.

Using isolated yeast cell membranes from MY1186 and FID, peptides of the present invention which blocked the interaction of Ras with mammalian GAP, were shown to also inhibit Ras-dependent yeast adenylyl cyclase activity. As a control for demonstrating the specificity of the peptides of the present invention, isolated cell membranes from yeast strain JC377-10B were also used in the adenylyl cyclase assay. The results are shown in Table VIII.

**TABLE VIII**

Effect of Peptides on Yeast Adenylyl Cyclase Activity

| A Peptide | Concentration (µM) | %Inhibition of Adenylyl Cyclase Activity Ras Dependent | Ras Independent |
|---|---|---|---|
| Ras 17-37 | 40 | 58 ± 2 | 0 |
| Ras 17-32 | 19 | 34 | 3 |
| Ras 23-37 | 100 | 2 | 6 |
| B Ras 17-32 | 10 | 26 ± 2 | ND* |
| Ras 23-37 | 10 | 5 ± 1 | ND |
| Ras 60-76 (SEQ ID NO:13) | 10 | 6 ± 1 | ND |
| Ras 19-33(1) (SEQ ID NO:5) | 100 | 37 | ND |
| Ras 19-33(2) (SEQ ID NO:6) | 100 | 21 | ND |

*ND—Not determined

Ras peptides were tested at the indicated concentrations for inhibition of S. cerevisiae adenylyl cyclase activity. Part A of Table VIII shows Ras-dependent adenylyl cyclase activity assayed with membranes from yeast strain MY1186 (expresses mammalian Ras). Ras-independent activity was assayed with membranes from strain JC377-10B (ras-minus, constitutively active adenylyl cyclase). Part B of Table VIII shows Ras-dependent adenylyl cyclase activity as assayed by reconstitution of membranes from strain FID (ras-minus) with 5 µM purified Ras protein.

The results of the yeast adenylyl cyclase assay demonstrate the ability of peptides Ras 17-37 and Ras 17-32 to inhibit MY1186 (mammalian Ras containing) adenylyl cyclase activity while peptide Ras 23-37 had no inhibitory effect. These peptides did not inhibit JC377-10B activity demonstrating the specificity of the peptides. Peptides Ras 17-32, Ras 19-33(1)(SEQ ID NO:5) and Ras 19-33(2) (SEQ ID NO:6) inhibited FID (no endogenous Ras) adenylyl cyclase activity when Ras was added to the membranes. The results of the yeast adenylyl cyclase inhibitor assay further demonstrates the ability of the peptides of the present invention, to specifically inhibit Ras function.

The biological activity of the synthetic peptides of the present invention as inhibitors of Ras function was determined in Xenopus oocyte germinal vessicle breakdown (GVBD) assays. Because Xenopus, oocytes respond to Ras microinjection by inducing GVBD (a widely accepted model of transformation), peptides that block Ras-GAP interactions can be tested in this system for their ability to inhibit Ras-induced transformation. GVBD causes the appearance of a white spot at the animal hemisphere of the oocyte.

[Val-12]Ras, which is known to cause GVBD in Xenopus oocytes, was injected into oocytes, as described in Example XI. The level of GVBD observed using only [Val-12]Ras was considered to be 100% and the reduction of this response was considered percent inhibition. As a control for demonstrating inhibition of Ras transformation, Ras neutralizing antibody Y13-259 was injected prior to, or coinjected with, [Val-12]Ras. The results of the Xenopus oocyte microinjection assay using Ras peptides Ras 19-33(2) (SEQ ID NO:6) and Ras 91-103 (SEQ ID NO: 14)of the present invention at a concentration of 4 mM, are shown in Table XI. The peptides were injected immediately prior to the injection of [Val-12]Ras.

## TABLE XI

### Inhibition of Xenopus oocyte Germinal Vessicle Breakdown

| Compound Injected | %Inhibition of [Val-12]Ras induced GVBD |
|---|---|
| Buffer | 0 |
| Y13-259 | 75 ± 6 |
| Ras 19-33(2) (SEQ ID NO:6) | 72 ± 5 |
| Ras 91-103 (SEQ ID NO:14) | 2 |

The results of this assay, as shown in Table XI demonstrate the ability of peptide Ras 19-33(2) (SEQ ID NO:6) of the present invention, to inhibit Ras induced transformation as measured by Xenopus oocyte GVBD. Ras 19-33(2) (SEQ ID NO:6) did not inhibit progesterone-mediated GVBD (which is Ras independent) indicating that Ras 19-33(2) (SEQ ID NO:6) is not cytotoxic. It is expected that Ras peptides Ras 17-44, Ras 17-37, Ras 17-32, Ras 19-31, Ras 19-31(1) (SEQ ID NO:3), Ras 19-32, Ras 20-32, Ras 21-37, Ras 26-32, Ras 19-32(D-Asn) (SEQ ID NO:4), Ras 19-33, Ras 19-33(1)(SEQ ID NO:5), Ras 19-33(3)(SEQ ID NO:7), Ras 19-33(4)(SEQ ID NO:8), Ras 19-33(5)(SEQ ID NO:9), Ras 19-33(6) (SEQ ID NO:10), Ras 19-33(7) (SEQ ID NO:11), and GAP888-910 (SEQ ID NO:2), of the present invention will have silimar activity in the Xenopus oocyte GVBD assay, as peptide Ras 19-33(2)(SEQ ID NO:6), since these peptides all demonstrated inhibition of Ras-GAP interaction (Table IV) and yeast adenylyl cyclase activity (Table VIII). Variation in the activity of these peptides is expected, however, due to differences in solubility and protease susceptibility.

The following examples illustrate the present invention without, however, limiting the same thereto. The disclosure of each reference mentioned in the following examples is hereby incorporated by reference.

EXAMPLE I

**Purification of bovine brain GAP**

All steps were carried out at 4°C. Fresh bovine brains were obtained from a local slaughterhouse. The cerebra were isolated and washed in ice-cold 0.9% NaCl. The cerebra from four to six animals were homogenized in a Waring blender in 3 liters of buffer A (25 mM TRIS-HCl, pH 7.5, 1 mM MgCl$_2$, 1 mM EGTA, 1mM dithiothreitol, 1 mM phenylmethylsulfonyl flouride). The homogenate was passed through five layers of cheesecloth and then centrifuged at 5000 x g for 20 minutes. The resulting supernatant was then centrifuged at 100,000 x g for 15 minutes to obtain a clarified high speed supernatant fraction.

The high-speed supernatant fraction was incubated as one batch, with 400 mL of DEAE-Sephacel (Pharmacia LKB) for at least 2 hours, and then washed using a Buchner funnel with 2 Liters of buffer A. The resin was poured into a 5 cm x 30 cm column and washed with an additional 1 Liter of buffer A. Elution was achieved with a 2 Liter, linear 0.0-0.3 M NaCl gradient in buffer A. The flow rate was 60 mL/hr, and 20 mL fractions were collected. A single symmetrical peak of GAP activity eluted between 70 and 90 mM NaCl, as determined by conductivity (model 32 conductance meter, Yellow Springs Instrument). The pooled peak fractions (200-300 mL) were concentrated to 30 mL by filtration with an Amicon PM-30 membrane in a stirred cell apparatus.

The concentrated pool was applied to two 5 cm diameter columns linked in tandem (total resin length, 150 cm) containing Sepharose 6B (Pharmacia LKB). Elution was continued at 60 mL/hr with buffer A, and 17.5 mL fractions were collected. A single peak of GAP activity eluted at 1.8 Liters of buffer A. For reference as standards, the proteins alcohol dehydrogenase (150 kDa) and bovine serum albumin (66 kDa) eluted at 1.78 and 1.90 Liters, respectively.

The pooled Sepharose 6B GAP eluate was applied to a 2.5 x 30 cm column of orange dye matrix (Amicon). The column was then washed with buffer A until the absorbance at 280 nm reached baseline. Elution was achieved with a 400 mL linear 0-0.4 M NaCl gradient in buffer A at 30 mL per hour and 3 mL fractions were collected. A single peak of GAP activity was detected between 40 and 70 mM NaCl.

Peak fractions from the orange dye matrix column (30-40 mL) were applied to a 1.5 x 27 cm column of green dye matrix (Amicon). After application, the column was washed in buffer A containing 20% (vol/vol) glycerol and 0.3 M NaCl. Elution of GAP activity was achieved with a 400 mL linear 0.3 M to 1.0 M NaCl gradient in buffer A plus 20% glycerol. Fractions (2 mL) were collected at 15 mL per hour. GAP activity was detected in a broad peak between 600 and 620 mM NaCl. The peak activity fractions (20-60 mL) were pooled and dialyzed overnight against 1 Liter of buffer A plus 20% glycerol, but lacking the phenylmethylsulfonyl fluoride.

The dialyzed material was applied to a Mono Q HR 5/5 column (Pharmacia LKB) at 0.7 mL per minute. Reservoir A contained 25 mM TRIS-HCl, pH 7.5; 1 mM EGTA; 1 mM MgCl$_2$; 1 mM dithiothreitol; 10% glycerol, and reservoir B contained the same buffer plus 1.0 M NaCl. Elution was achieved with an increasing NaCl gradient from 0 to 70 mM for 5 minutes, 70 mM to 150 mM for 30 minutes, and 150 mM to 1000 mM for 10 minutes. The flow rate was 0.7 mL per minute and 0.5 mL fractions were collected. A single peak of GAP activity, corresponding to a single peak of A$_{280}$ absorbing material, eluted at about 120 mM NaCl. The fractions were stored on ice, at 4°C. Activity was more stable at 4°C upon the addition of bovine serum albumin (0.5 mg/mL). Dilutions of GAP prior to assay were done with buffer containing 25 mM TRIS-HCl, pH7.5, 1 mM EGTA, 1 mM MgCl$_2$, 1 mM DTT, 1 mg/mL BSA.

## EXAMPLE II

### Recombinant expression of GAP in E. coli

The bovine GAP cDNA clone lambda GAPI is known in the art [Vogel, U. S., et al., (1988) Nature, 335, pp90-93) as is the CO$_2$H-terminal GAP cDNA expression plasmid [Marshall, M. S., (1989) EMBO J., 8, pp1105-1110).

A bacterial expression plasmid was constructed to express full length bovine GAP. The bacterial expression vector pUC19 was digested with EcoRI and SalI. The 3,721 base pair (bp) cDNA encoding bovine GAP was removed from Zap³ by digestion with EcoRI and NotI. Zap³ is a 95% complete full length bovine GAP cDNA as an EcoRI fragment in vector Lambda-Zap (Stratagene, La Jolla, California). The NotI-EcoRI GAP-cDNA fragment was ligated into the SalI-EcoRI digested pUC19, together with a synthetic, 95 bp DNA oligonucleotide. This synthetic oligonucleotide encodes, in their respective order, a 5' SalI site, a bacterial and yeast ribosome binding site, the first 23 amino acids of bovine GAP, and a 3' NotI site. The primary structure of the synthetic oligonecleotide is:

```
TCGACGAATT CAAGGAGGAA AAACAAAATG ATGGCGGCCG   40
AGGCCGGCGG TGAGGAGGGC GGCCCGGTCA CCGCCGGGGC   80
AGCGGGAGGC GGCGC (SEQ ID NO:16).             95
```

This plasmid was designated pYO, and expresses full length bovine GAP.

## EXAMPLE III

### Purification of recombinant GAP from E. coli

GAP proteins were expressed in E. coli strain JM109 by overnight growth in Luria broth with 50 μM ampicillin

and 100 µM isopropyl thio -β-D-galactoside (IPTG). The cell paste from 6 liters of culture was lysed at 4°C in 90 mL of buffer B, consisting of 50 mM TRIS-HCl, pH 7.5, 1 mM EDTA, 5 mM DTT, 200 µM PMSF, 10 µg per mL aprotinin, 1 µg per mL leupeptin, 1 µg per mL antipain, 10 µg per mL epoxypropyl-L-leucylamido-(4-guanidino)-butane, and 10 µg per mL benzamidine. To this was added 200 µg per mL lysozyme, and incubated on ice for 20 minutes. Lubrol-PX (Sigma) was then added, to a final concentration of 0.1% and incubated on ice for five minutes. To eliminate high molecular weight DNA, the lysate was incubated with 10 mM $MgCl_2$ and 10 µg per mL of DNase I. Buffer B was used for all subsequent steps. After centrifugation at 10,000 x g for 15 minutes, the supernatant fraction was applied to a column (2.5 cm x 30 cm) of DEAE-sephacel (Pharmacia-LKB) and the column was washed with buffer B until the absorbance at 280 nm reached the baseline. GAP was eluted from the column using 600 mL of a linear 0-0.45 M NaCl gradient in buffer B, at a flow rate of 10 mL per hour, and collecting 4 mL fractions. Full length GAP eluted between about 74 mM and 110 mM NaCl.

## EXAMPLE IV

### GAP Detection By Immunoblot

The cell pellet from a 10 mL culture of E. coli expressing GAP protein was resuspended in 0.5 mL of buffer C (50 mM TRIS-HCl, pH7.5, 2 mM EDTA, 1 mM DTT, 200 µM PMSF, and 1% aprotinin) and lysed as described in Example III. The lysate was then centrifuged at 7,000 x g for five minutes. The pellet was dissolved in 0.5 mL of buffer C. The total protein concentration was determined by the coomassie blue dye method. About 30 µg of protein was electrophorsed at 25 mA on a 13.5% polyacrylamide gel containing 0.1% SDS, using a Bio-Rad "baby" gel apparatus. Following electophoresis, the gel was washed for 15 minutes in transfer buffer (25 mM TRIS-base, 192 mM glycine and 20% methanol) followed by electophoretic transfer at 4°C into nitrocellulose paper at 24 V for 15 hours, then 250 mA for one hour. The nitrocellulose paper was then incubated in a buffer consisting of 25 mM TRIS-HCl, pH 7.5, 150 mM NaCl, 0.4% powdered milk, 0.5% bovine serum albumin, 1 mM $CaCl_2$ and 20 µM EDTA, for 30 minutes at 4°C. The nitrocellulose paper was then incubated for about 14 hours at 4°C with rabbit anti-GAP antiserum (rabbit antiserum 638 for detection of full length GAP. [Vogel, U.S. et al. 1988), Nature, 335 pp.90-93].

The nitrocellulose paper was then washed with wash buffer (25 mM TRIS-HCl, pH 7.5, 150 mM NaCl, and 0.1% Tween 20), followed by incubation with $^{125}$I-labeled goat anti-rabbit antibody. The nitrocellulose paper was then washed with wash buffer and used to expose Kodak-XAR-2 film at -70°C.

## EXAMPLE V

### Recombinant Ha-ras protein purification

E. coli (strain RRIIaci[q]) containing the expression plasmid pRas[Gly[12] Ala[59]] [Gibbs, J. et al., (1984), PNAS, 81, pp. 5705-5708, were grown in 1 Liter cultures in Luria broth for 20 hour at 37°C in the presence of 0.1 mM isopropyl thio-β-D-galactoside (Bethesda Research Laboratories) and ampicillin at 50 µg/mL. Bacteria were lysed in buffer C with lysozyme (Sigma) at 100 µg/mL and 0.01% Nonidet P-40 (Sigma). The prepared lysate was applied to a column (2.5 cm x 90 cm) of Sephadex G-75 superfine (Pharmacia LKB) and chromatographed at 10 mL per hour in buffer containing 50 mM TRIS-HCl, pH 7.5, 1 mM EDTA, and 1 mM DTT. Three mL fractions were collected. The p21 that eluted from the Sephadex G-75 column was greater than 90% homogeneous as analyzed by $NaDodSO_4$/polyacrylamide gel electrophoresis on 13.5% polyacrylamide gels. About 10 mg of purified protein was readily obtained from a 1 Liter bacterial culture. During purification, p21 elution was followed by $NaDodSO_4$/polyacrylamide gel electrophoresis, or W absorbance at 280 nm.

## EXAMPLE VI

### Guanine Nucleotide exchange of Ras

To prepare a substrate for the GAP assay, normal Ha-ras p21 was equilibrated with [γ-$^{32}$P]GTP (3000 Ci/mmol, ICN Radiochemicals), in the following reaction: 0.1 nmol of Ha-ras p21 and 0.2 nmol [γ-$^{32}$P]GTP were added to 1 mL of buffer containing 2 mM EDTA, 2 mM DTT, 100 µg/mL BSA (Sigma: fatty acid free), 25 mM TRIS-HCl, pH 7.5. Following incubation for 15 minutes at 30°C, the reaction was chilled on ice and applied to a PD-10 column (Pharmacia) to remove free guanine nucleotide. The RAS-GTP was eluted from the column at 4°C by adding successive 1 mL aliquots of buffer D (1 mM $MgCl_2$, 20 mM sodium HEPES, pH 7.5), and collecting 1 mL column fractions. Aliquots of each fraction were analyzed by a filter binding assay to determine the efficiency of the guanine nucleotide exchange reaction [Gibbs, J., et al., (1984) PNAS, 81, pp. 5705-5708]. Only fractions having greater than 80% of the [γ-32P]GTP incorporated into ras p21 were used in the GAP assays.

## EXAMPLE VII

### GAP Assays

Ras GTPase activity was measured by the charcoal adsorption procedure. Each 50 μL reaction contained 10 fmol purified GAP, 240 fmol Ha ras p21-[γ $^{32}$P] GTP and 1 mg/mL BSA in buffer D. Varying amounts of the competing peptides were added as indicated. Prewarmed reactions (24°C) were initiated by the addition of GAP, then incubated for 4 minutes at 24°C. The reactions were terminated by the addition of 5 volumes of ice cold 5% activated charcoal (Sigma: HCl washed) in 50 mM NaH$_2$PO$_4$. GTPase activity was quantitated by measuring the free $^{32}$P-orthophosphate in the cleared supernatant after centrifugation of the charcoal mix. GAP-stimulated Ras GTPase activity in the absence of competitors was 40-60 fmol Ras-GTP hydrolyzed; the 100% control value. In the absence of GAP, GTP hydrolysis was not detectable during the incubation period of the assay. Inhibitors of this assay block GAP-stimulated Ras GTPase without impairing intrinsic Ras GTPase, assayed in the absence of GAP following incubation at 24°C for one hour.

## EXAMPLE VIII

### Binding of Ras peptides to GAP

Ras peptides were dissolved in either 100% dimethyl formamide (DMF, American Burdick and Jackson) or 100% dimethyl sulfoxide (DMSO, Fluka UV-spectroscopy grade) at a calculated peptide concentration of 1-4 mM. Following 1 minute in a Sonicor SC-200 bath sonicator at room temperature, the peptide solutions were vortexed, and then centrifuged for 5 minutes at room temperature in an Eppendorf microfuge to remove any undissolved material. 20 μL of the cleared peptide solutions were then added to 80 μL of Buffer D in a dilution series covering a range of peptide concentrations from 0.02 μM up to the maximum solubility limit for each peptide. In each dilution, the solvent concentration was maintained at 20%. These peptides solutions were then centrifuged for 5 minutes at room temperature at 10,000 x g, and the cleared supernatants were divided into 2 parts. One part of each cleared peptide dilution was used in a GAP competition assay: 40 μL was added to a 5 μL solution containing 0.1 mg/mL BSA plus 240 fmol Ha-ras p21-[γ-$^{32}$P]GTP. Reactions were initiated upon addition of 5 μL of diluted GAP (10 fmol), and analyzed as described above.

The other portion of the cleared peptide solution was measured spectrophotometrically to determine solubility and concentration of the peptides. Extinction coefficients were determined by measuring the absorbance at 277 nm of 2 peptides whose exact concentrations were quantitated by amino acid analysis. The extinction coefficients for peptide Ras 17-37 dissolved in buffer D/20% DMF or peptide Ras 91-103 (SEQ ID NO:14)dissolved in water/20% DMSO, were 2580 cm$^{-1}$M$^{-1}$ and 2230 cm$^{-1}$ M$^{-1}$, respectively. These values were used to determine the exact concentration for each peptide used at each point in the competition assay. In addition, peptide solubility was confirmed by comparing the light scattering at 600 nm for each peptide solution measured before and after centrifugation. Only concentrations reflecting 100% solubility were used in the dose response curves, and the maximum concentration used for each peptide competitor indicates the limit of solubility for that peptide.

## EXAMPLE IX

### Peptide Synthesis

The peptides were prepared by solid phase peptide synthesis chemistry following the method of Merrifield, J.Am. Chem. Soc. 85: pp.2149-2154, using a double-coupling protocol for the introduction of all amino acids on the Applied Biosystems automated peptide synthesizer (model 430A). The synthesis procedure is described in detail in the Applied Biosystems model 420A Peptide Synthesizer Operator's Manual. Deprotection and cleavage of the peptide from the resin support was accomplished by treatment with hydrogen flouride. The peptides were purified by preparative HPLC on a reverse-phase C$_{18}$ silica column (25 cm x 2.5 in) using a 0% to 100% gradient of acetonitrile containing 0.1% trifluoroacetic acid. Peptide homogeneity was demonstrated by analytical HPLC, and peptide identity was confirmed by amino acid analysis, and mass spectroscopy.

EXAMPLE X

**Adenylyl Cyclase Assay**

Yeast strain FID, MY1186 and JC377-10B, were grown in YEHD medium at 30°C. Membrane fractions were prepared from cultures grown to an $A_{660}$ of 0.5 to 1.0 by glusulase digestion [Casperson, G.F. et al., (1983), J. Biol. Chem., 258, pp. 7911-7914], Dounce homogenization of the spheroplasts in buffer containing 50 mM sodium MES [2(N-morpholino) ethanesulfonic acid] (pH 6.0), 0.1 mM EGTA [ethylene glycol-bis($\beta$-aminoethyl ether)-N, N, N′, N′-tetraacetic acid], 0.1 mM $MgCl_2$, 1 mM phenylmethylsulfonyl fluoride, 1mM dithiothreitol, and then centrifugation at 105,000 x g for 30 minutes at 4°C. Adenylyl cyclase assays (100-$\mu$L reaction volume) measured the conversion of [$\alpha$-$^{32}$P]ATP to [$^{32}$P]cAMP at 30°C, using 50 $\mu$g to 100 $\mu$g of membrane protein in assays using membranes from yeast strains MY1186 and JC377-10B.. Product was separated from reactants by sequential column chromatography on Dowex AG 50W-X4 (200 to 400 mesh; Bio-Rad) and neutral alumina (Sigma, type WN-3). Other assays used membranes prepared from strain FID. Since these membranes contain adenylyl cyclase, but are lacking Ras protein, stimulation of adenylyl cyclase activity may be measured following reconstitution with purified Ras complexed with GTP. Reactions containing 83 $\mu$g of FID membranes and ras, proteins at a final concentration of 5 to 20 $\mu$M were carried out in a 100 $\mu$L volume according to the procedure described by Casperson et al.

Statistical estimates are expressed as the mean ± SEM, for results from three or more experiments.

EXAMPLE XI

**Xenopus oocyte microinjection**

Microinjection of frog oocytes was done according to the method of Gibbs, J.B. et al., (1989) P.N.A.S. USA, 86, pp.6630-6634. Large (10-12 cm) female Xenopus laevis frogs were obtained from Nasco (Fort Atkinson, WI); the frogs were treated with chorionic gonadotropin by the supplier. After hypothermic anaestheisa, frogs were sacrificed to obtain oocytes.

Oocytes were incubated, rocking at room temperature, with 2 mg of collagenase (type I; Sigma Chemical Co.) per mL in calcium-free MBSH (88 mM NaCl, 1 mM KCl, 0.82 mM $MgSO_4$, 2.4 mM $NaHCO_3$, 10 mM HEPES [N-2-hydroxyethylpiperazine-N′-2-ethanesulfonic acid], pH 7.4) plus 0.1 mg of gentamicin sulfate (GIBCO Laboratories) per mL for one hour with two changes of the collagenase solution and occasional gentle pipetting. Oocytes were then placed in MBSH medium containing 0.4 mM $CaCl_2$ and 0.33 mM $Ca(NO_3)_2$.

After resting the oocytes for 4-16 hours, groups of 20-40 stage V-VI oocytes were injected with approximately 50 nL of test solution per oocyte using a Medical Systems model PLI 100 injector (Greenvale, NY). After incubation at 19°C for 21 hours, germinal vessicle breakdown was evaluated by the appearance of a white spot in the animal hemisphere of the oocyte.

For induction of GVBD, [Val-12]Ras was injected a 1 mg/mL. Progesterone was added at 100 nM as a control for the ability of the oocytes to undergo GVBD by a Ras-independent mechanism. Neutralizing antibody Y13-259 was injected at 3 mg/mL with [Val-12]Ras as a control for inhibition of Ras induced GVBD. Both peptides Ras 19-33(2) (SEQ ID NO:6) and Ras 91-103 (SEQ ID NO:14), dissolved in water, were injected at 4mM. Ras 91-103 (SEQ iD NO: 14) is non-inhibitory in the GAP assay.

Statistical estimates are expressed as the mean ± SEM, for results from three or more experiments.

## SEQUENCE LISTING

(2) INFORMATION FOR SEQ ID NO:   1
    Ser Ala Leu Thr Ile Gln Leu Ile Gln Asn His
    1               5                   10
    Phe Val Asp Glu Tyr Asp Pro Thr Ile Glu Asp Ser
                    15                  20
    Tyr Arg Lys Gln Val.
            25


    (i)   SEQUENCE CHARACTERISTICS:
          (A)   LENGTH:   28 Amino Acids
          (B)   TYPE:   AMINO ACID
          (D)   TOPOLOGY:   Linear
    (ii) MOLECULE TYPE: Peptide
    (xi) SEQUENCE DESCRIPTION: SEQ ID NO:1:
(2) INFORMATION FOR SEQ ID NO:   2
    (i)   SEQUENCE CHARACTERISTICS:
          (A)   LENGTH:   23 Amino Acids
          (B)   TYPE:   Amino Acid
          (D)   TOPOLOGY:   Linear
    (ii) MOLECULE TYPE: Peptide
    (xi) SEQUENCE DESCRIPTION: SEQ ID NO:2:
    Met Arg Thr Arg Val Val Ser Gly Phe Val Phe Leu
    1               5                   10
    Arg Leu Ile Gly Pro Ala Ile Leu Asn Pro Arg.
                    15                  20

(2) INFORMATION FOR SEQ ID NO: 3
    (i) SEQUENCE CHARACTERISTICS:
        (A) LENGTH: 12 Amino Acids
        (B) TYPE: Amino Acid
        (D) TOPOLOGY: Linear
    (ii) MOLECULE TYPE: Peptide
    (xi) SEQUENCE DESCRIPTION: SEQ ID NO:3:
Leu Thr Ile Gln Ile Gln Asn His
1               5
Phe Val Asp Glu
     10

(2) INFORMATION FOR SEQ ID NO: 4
    (i) SEQUENCE CHARACTERISTICS:
        (A) LENGTH: 14 Amino Acids
        (B) TYPE: Amino Acids
        (D) TOPOLOGY: Linear
    (ii) MOLECULE TYPE: Peptide
    (ix) FEATURE:
        (A) NAME/KEY: Xaa
        (B) LOCATION: Amino Acid 8
        (D) OTHER INFORMATION: Xaa is the D isomer
                      of Asn.
    (xi) SEQUENCE DESCRIPTION: SEQ ID NO:4:
        Leu Thr Ile Gln Leu Ile
        1               5
        Gln Xaa His Phe Val Asp Glu Tyr
            10

(2)  INFORMATION FOR SEQ ID NO:   5

    (i)  SEQUENCE CHARACTERISTICS:

        (A)  LENGTH:   17 Amino Acids

        (B)  TYPE:   Amino Acid

        (D)  TOPOLOGY:   Linear

    (ii) MOLECULE TYPE: Peptide

    (ix) FEATURE:

        (A)  NAME/KEY:   Xaa

        (B)  LOCATION:   Amino Acid 17

        (D)  OTHER INFORMATION:   Xaa is Asp with the
                                   alpha amino group
                                   substituted with an
                                   acetyl group.

    (xi) SEQUENCE DESCRIPTION: SEQ ID NO:5:

        Arg Arg Leu Thr Ile Gln Leu Ile
        1               5
        Gln Asn His Phe Val Asp Glu Tyr Xaa
          10                  15

(2) INFORMATION FOR SEQ ID NO:  6

    (i)    SEQUENCE CHARACTERISTICS:

        (A)    LENGTH:  17 Amino Acids

        (B)    TYPE:  Amino Acid

        (D)    TOPOLOGY:  Linear

    (ii) MOLECULE TYPE: Peptide

    (ix) FEATURE:

        (A)    NAME/KEY:  Xaa(1)

        (B)    LOCATION:  Amino Acid 1

        (D)    OTHER INFORMATION:  Xaa(1) is Arg with an acetyl group substituted for the alpha amino group.

    (ix) FEATURE:

        (A)    NAME/KEY:  Xaa(2)

        (B)    LOCATION:  Amino Acid 17

        (D)    OTHER INFORMATION:  Xaa(2) is Asp with an amido group substituted for the alpha carboxy group.

    (xi) SEQUENCE DESCRIPTION: SEQ ID NO:6:

        Xaa(1) Arg Leu Thr Ile Gln Leu Ile
        1                 5

        Gln Asn His Phe Val Asp Glu Tyr Xaa(2)
           10                    15

(2) INFORMATION FOR SEQ ID NO:  7

    (i)    SEQUENCE CHARACTERISTICS:

        (A)    LENGTH:  17 Amino Acids

        (B)    TYPE:  Amino Acid

        (D)    TOPOLOGY:  Linear

    (ii) MOLECULE TYPE: Peptide

    (ix) FEATURE:

        (A)    NAME/KEY:  Xaa

        (B)    LOCATION:  Amino Acid 17

        (D)    OTHER INFORMATION:  Xaa is Asp with the alpha carboxy group substituted with an amido group.

    (xi) SEQUENCE DESCRIPTION: SEQ ID NO: 7:

Glu Glu Leu Thr Ile Gln Leu Ile
1               5

Gln Asn His Phe Val Asp Glu Tyr Xaa
  10                    15


(2) INFORMATION FOR SEQ ID NO:  8

    (i)    SEQUENCE CHARACTERISTICS:

        (A)    LENGTH:  17 Amino Acids

        (B)    TYPE:  Amino Acid

        (D)    TOPOLOGY:  Linear

    (ii) MOLECULE TYPE: Peptide

    (xi) SEQUENCE DESCRIPTION: SEQ ID NO:8:

Arg Arg Leu Thr Ile Gln Leu Ile
1               5

Gln Asn His Phe Val Asp Glu Tyr Asp
  10                    15

(2) INFORMATION FOR SEQ ID NO:  9

    (i)   SEQUENCE CHARACTERISTICS:

        (A)   LENGTH:  16 Amino Acids

        (B)   TYPE:  Amino Acid

        (D)   TOPOLOGY:  Linear

    (ii) MOLECULE TYPE: Peptide

    (xi) SEQUENCE DESCRIPTION: SEQ ID NO:9:

             Arg Arg Leu Thr Ile Gln Leu Ile
             1            5

             Gln His Phe Val Asp Glu Tyr Asp
               10           15


(2) INFORMATION FOR SEQ ID NO:  10

    (i)   SEQUENCE CHARACTERISTICS:

        (A)   LENGTH:  17 Amino Acids

        (B)   TYPE:  Amino Acid

        (D)   TOPOLOGY:  Linear

    (ii) MOLECULE TYPE: Peptide

    (xi) SEQUENCE DESCRIPTION: SEQ ID NO:10:

             Arg Ala Leu Thr Ile Gln Leu Ile
             1            5

             Gln Asn His Phe Val Asp Glu Tyr Asp
               10           15

(2) INFORMATION FOR SEQ ID NO: _11_

    (i)   SEQUENCE CHARACTERISTICS:

        (A)  LENGTH: _16 Amino Acids_

        (B)  TYPE: _Amino Acid_

        (D)  TOPOLOGY: _Linear_

    (ii) MOLECULE TYPE: Peptide

    (ix) FEATURE:

        (A)  NAME/KEY: _Xaa_

        (B)  LOCATION: _Amino Acid 16_

        (D)  OTHER INFORMATION: _Xaa is Asp with the_
                                        _alpha carboxy group_
                                        _substituted with an_
                                        _amido group._

    (xi) SEQUENCE DESCRIPTION: SEQ ID NO:11:

        Ser Arg Thr Ile Gln Leu Ile Gln
        1          5

        Asn His Phe Val Asp Glu Tyr Xaa
          10                15


(2) INFORMATION FOR SEQ ID NO: _12_

    (i)   SEQUENCE CHARACTERISTICS:

        (A)  LENGTH: _16 Amino Acids_

        (B)  TYPE: _Amino Acid_

        (D)  TOPOLOGY: _Linear_

    (ii) MOLECULE TYPE: Peptide

    (xi) SEQUENCE DESCRIPTION: SEQ ID NO:12:

        Tyr Arg Lys Gln Val Val Ile Asp
        1          5

        GLY Glu Thr Ser Leu Leu Asp Ile
          10                15

(2) INFORMATION FOR SEQ ID NO: 13

    (i) SEQUENCE CHARACTERISTICS:

        (A) LENGTH: 17 Amino Acids

        (B) TYPE: Amino Acid

        (D) TOPOLOGY: Linear

    (ii) MOLECULE TYPE: Peptide

    (xi) SEQUENCE DESCRIPTION: SEQ ID NO:13:

        Gly Gln Glu Glu Tyr Ser Ala Met
        1          5

        Arg Asp Gln Tyr Met Arg Thr Gly Glu
          10          15


(2) INFORMATION FOR SEQ ID NO: 14

    (i) SEQUENCE CHARACTERISTICS:

        (A) LENGTH: 13 Amino Acids

        (B) TYPE: Amino Acid

        (D) TOPOLOGY: Linear

    (ii) MOLECULE TYPE: Peptide

    (xi) SEQUENCE DESCRIPTION: SEQ ID NO:14:

        Glu Asp Ile His Gln Tyr Arg Glu
        1          5

        Gln Ile Lys Arg Val
          10

(2) INFORMATION FOR SEQ ID NO: __15__

    (i)    SEQUENCE CHARACTERISTICS:

        (A)   LENGTH: __12 Amino Acids__

        (B)   TYPE: __Amino Acid__

        (D)   TOPOLOGY: __Linear__

    (ii) MOLECULE TYPE: Peptide

    (xi) SEQUENCE DESCRIPTION: SEQ ID NO:15:

        Glu Ser Arg Gln Ala Gln Asp Leu

        1               5

        Ala Arg Ser Tyr

          10


(2) INFORMATION FOR SEQ ID NO: __16__

    (i)    SEQUENCE CHARACTERISTICS:

        (A)   LENGTH: __95 base pairs__

        (B)   TYPE: __DNA__

        (C)   STRANDEDNESS: __Double__

        (D)   TOPOLOGY: __Linear__

    (ii) MOLECULE TYPE: Other nucleic acid (DNA)

    (xi) SEQUENCE DESCRIPTION: SEQ ID NO:16:

        TCGACGAATT CAAGGAGGAA AAACAAAATG ATGGCGGCCG  40

        AGGCCGGCGG TGAGGAGGGC GGCCCGGTCA CCGCCGGGGC  80

        AGCGGGAGGC GGCGC.                        95


**Claims**

1.    A peptide in substantially pure form which can bind to GTPase activating protein, and inhibit Ras activation by GTPase activating protein.

2.    The peptide according to Claim 1 which inhibits Ras dependent cell transformation.

3.    A compound in substantially pure form having an amino acid sequence which corresponds to the region of the Harvey Ras protein, between amino acid 17 and amino acid 44 of Harvey Ras, which binds to GTPase activating protein and inhibits Ras activation by GTPase activating protein.

4.    A compound in substantially pure form having the formula:

```
Ser Ala Leu Thr Ile Gln Leu Ile Gln Asn His Phe Val
1               5                   10
Asp Glu Tyr Asp Pro Thr Ile Glu Asp Ser Tyr Arg Lys
      15                  20                      25
Gln Val (SEQ ID NO:1).
```

5. A compound in substantially pure form having the formula:

```
Ser Ala Leu Thr Ile Gln Leu Ile Gln Asn His Phe Val
1               5                   10
Asp Glu Tyr Asp Pro Thr Ile Glu; or
      15                  20
```

```
Ser Ala Leu Thr Ile Gln Leu Ile Gln Asn His Phe Val
1               5                   10
Asp Glu Tyr; or
      15
Leu Thr Ile Gln Leu Ile Gln Asn His Phe Val Asp Glu
1               5                   10
Tyr ; or
```

```
Leu Thr Ile Gln Leu Ile Gln Asn His Phe Val Asp Glu
1               5                   10
Tyr Asp ; or
      15
```

```
Ile Gln Leu Ile Gln Asn His Phe Val Asp Glu Tyr.
1               5                   10
```

6. A compound in substantially pure form having the formula:

```
Asn His Phe Val Asp Glu Tyr.
1               5
```

7. A compound in substantially pure form having the formula:

```
Leu Thr Ile Gln Leu Ile Gln D-Asn His Phe Val Asp
.1              5                       10
Glu Tyr (SEQ ID NO:4).
```

8. A compound in substantially pure form having the formula:

```
Arg Arg Leu Thr Ile Gln Leu Ile Gln Asn His Phe Val
1               5                   10
Asp Glu Tyr Asp-NH2 (SEQ ID NO:5).
    15
```

9. A compound in substantially pure form having the formula:

```
         O
         ||
CH3-C-Arg Arg Leu Thr Ile Gln Leu Ile Gln Asn His Phe
      1                5                   10
Val Asp Glu Tyr Asp-NH2 (SEQ ID NO:6).
        15
```

10. A compound in substantially pure form having the formula:

```
Glu Glu Leu Thr Ile Gln Leu Ile Gln Asn His Phe Val
1               5                   10
Asp Glu Tyr Asp NH2(SEQIDNO:7).
    15
```

11. A compound in substantially pure form which corresponds to the region of GTPase activating protein, which binds to Ras protein and inhibits Ras activation by GTPase activating protein.

12. The compound according to Claim 11 which inhibits Ras dependent cell transformation.

13. A compound in substantially pure form having the formula:

```
Met Arg Thr Arg Val Val Ser Gly Phe Val Phe Leu Arg
1               5                   10
Leu Ile Gly Pro Ala Ile Leu Asn Pro Arg (SEQ ID NO:2).
    15                  20
```

14. A compound in substantially pure form having the formula:

```
Z-R-Ser Ala Leu Thr Ile Gln Leu Ile Gln Asn His Phe
    1               5                   10
Val Asp Glu Tyr Asp Pro Thr Ile Glu Asp Ser Tyr Arg
        15                  20                  25
Lys Gln Val-R1 (SEQ ID NO:1)
```

wherein R is a peptidyl moiety of 1-6 amino acids which are Lys, Arg, His, Asp, Glu, or ornithine, and $R^1$ is amido, or mono-, or di- lower alkyl amido or OH, and Z is H, acetyl, or lower alkyl acyl, whenever lower alkyl is 1-6 carbon atoms.

15. The compound according to Claim 14 wherein the amino acids possess the natural L-configuration, and any optically active amino acid substituted by its D-isomer.

**16.** A compound in substantially pure form having the formula:

```
Arg Arg Leu Thr Ile Gln Leu Ile Gln Asn His Phe Val
1               5                   10
 Asp Glu Tyr Asp (SEQ ID NO:8);
     15


Arg Arg Leu Thr Ile Gln Leu Ile Gln His Phe Val Asp
1               5                   10
Glu Tyr Asp (SEQ ID NO:9);
    15


Leu Thr Ile Gln Leu Ile Gln Asn His Phe Val Asp Glu
1               5                   10
(SEQ ID NO:4);


Leu Thr Ile Gln Ile Gln Asn His Phe Val Asp Glu
1               5                   10
(SEQ ID NO:3);


Arg Ala Leu Thr Ile Gln Leu Ile Gln Asn His Phe Val
1               5                   10
Asp Glu Tyr Asp (SEQ ID NO:10) ; or
     15


Ser Arg Thr Ile Gln Leu Ile Gln Asn His Phe Val Asp
1               5                   10
Glu Tyr Asp-NH2 (SEQ ID NO:11)
```

**17.** A compound in substantially pure form having the formula:

EP 0 496 162 A2

```
Arg Leu Thr Ile Gln Leu Ile Gln Asn His Phe Val Asp
1               5                   10
Glu Tyr Asp;
        15
Arg Arg Leu Thr Ile Gln Leu Ile Gln His Phe Val Asp
1               5                   10
Glu Tyr Asp;


Leu Thr Ile Gln Leu Ile Gln Asn His Phe Val Asp Glu ;
1               5                   10

  or


Leu Thr Ile Gln Ile Gln Asn His Phe Val Asp Glu .
1               5                   10
```

18. A compound in substantially pure form having the formula:

```
R-Ala Leu Thr Ile Gln Leu Ile Gln Asn His Phe
      1               5                   10
Val Asp Glu Tyr Asp-R$^1$ (SEQ ID NO:10); or


Ser-R-Thr Ile Gln Leu Ile Gln Asn His Phe Val Asp
1               5                   10
Glu Tyr Asp-R$^1$ (SEQ ID NO.11),
        15
```

wherein R is a peptidyl moiety of 1 to 6 amino acids which are Lys, Arg, His, Asp, Glu, or ornithine, and $R^1$ is amido, or mono- or di-lower alkyl amido.

19. The compound according to Claim 18 wherein the amino acids possess the natural L-configuration, and any optically active amino acid substituted by its D-isomer.

31